# EUROPEAN PATENT SPECIFICATION

(11) **EP 2 704 724 B1**
(45) Date of publication and mention of the grant of the patent: **26.10.2016**
(21) Application number: 12719304.3
(22) Date of filing: 18.04.2012
(51) Int. Cl.: A61P 11/00

(54) **IMPROVED SUSPENSION FORMULATION OF BECLOMETASONE DIPROPIONATE FOR ADMINISTRATION BY INHALATION**
VERBESSERTE BECLOMETHASON-DIPROPIONAT SUSPENSIONSFORMULIERUNG ZUR VERABREICHUNG MITTELS INHALATION
FORMULATION DE SUSPENSION DE BECLOMETHASONE DIPROPIONATE AMÉLIORÉE À ADMINISTRER PAR INHALATION

(30) Priority: 03.05.2011 EP 11164575
(43) Date of publication of application: 12.03.2014
(60) Divisional application: 16178624.9
(73) Proprietor: Chiesi Farmaceutici S.p.A., 43100 Parma (IT)
(72) Inventor: CANTARELLI, Anna Maria, I-43100 Parma (IT); MINARI, Stefano, I-43100 Parma (IT); BASSI, Barbara, I-43100 Parma (IT)
(74) Representative: Minoja, Fabrizio
(86) International application number: PCT/EP2012/057068
(87) International publication number: WO 2012/150131

(56) References cited:
- WO-A1-03/086347
- WO-A1-2009/074666
- WO-A2-2005/115332
- US-A1- 2004 208 831

## Description

The present invention relates to a formulation in form of aqueous suspension of drug particles of a corticosteroid to be administered by nebulisation, characterised by an optimal particle size distribution.

The invention also relates to processes for preparing said formulation, uses and kits thereof.

### BACKGROUND OF THE INVENTION

The method of delivering drugs by inhalation has been used for several years, and is the mainstay of the treatment of diseases that limit the respiratory flow, such as asthma and chronic bronchitis.

The advantages of inhalation over the systemic route include the fact that the drug is released directly at the site of action, thus preventing systemic side effects and resulting in a more rapid clinical response and a higher therapeutic index.

Among the various types of drug which are administered by inhalation for the treatment of the respiratory diseases, insoluble or poorly water soluble corticosteroids, such as beclomethasone dipropionate (BDP), mometasone furoate, flunisolide, budesonide, fluticasone propionate and others are of great importance. Said drugs could be administered by nebulisation as micronised particles in suspension in an aqueous phase that usually also contains surfactants and/or other excipients.

The efficacy of this form of administration depends on the deposit of a sufficient quantity of particles at the site of action.

In order to ensure an effective penetration into the lower respiratory tract of the patient, i.e. bronchioli and alveoli, one of the most important parameters is particle size, which must be of some micron.

Particles with a larger diameter are ineffective because they are deposited in the oropharyngeal cavity, and are therefore unable to reach the terminal branches of the respiratory tree; they can also give rise to local side effects, or may be absorbed through the buccal mucosa and give rise to systemic side effects.

It is important to ensure correct administration, and therefore therapeutic efficacy, that a homogenous dispersion of the particles in suspension is achieved, without the formation of aggregates. Said aggregates can indeed prejudice the efficacy of nebulisation as particles with a diameter exceeding 5-6 µm are unable to reach the preferential site of action. The formation of aggregates, especially "cakes", i.e. sets of highly compact suspended particles, can also give rise to problems of distribution and therefore of uniformity of dose during the filling of the formulation in the containers, making the administration less therapeutically effective, as the dose may be transferred incompletely from the vial to the bulb of the nebuliser apparatus by the patient.

During the manufacturing of the formulation, it would also be useful incrementing the wettability of the drug in such a way as to increase its speed of dispersion in the aqueous phase, a step that is often time-consuming.

Further, a mandatory requirement that should be met by pharmaceutical formulations for nebulisation is sterility, as confirmed by the various Acts and Directives governing their quality and safety.

The current trend is to produce inhalation formulations devoid of preservatives and bacteriostatics and including the fewest possible excipients, as it has been reported in the literature that some of the substances commonly used in said formulations might induce allergic reactions or give rise to irritation of the respiratory mucosae.

On the other hand, the limited choice of excipients could make the task of preparing physically stable formulations.

All these problems have been solved by the formulation of the present invention characterised by a selected very narrow and well-defined particle size distribution of the suspended particles.

In WO 00/25746, WO 03/086347, and WO 2004/054545, the applicant has disclosed processes for the preparation of aqueous suspensions of corticosteroids for nebulisation, but said documents are silent about a particle size distribution of the suspended particles fulfilling the characteristics outlined and claimed as follows.

### SUMMARY OF THE INVENTION

In a first aspect, the present invention is directed to a propellant-free sterile pharmaceutical formulation for administration by nebulisation comprising micronized particles of beclometasone dipropionate monohydrate suspended in an aqueous solution at a concentration of 0.04% w/v, wherein i) no more than 10% of said suspended particles have a volume diameter [d(v,0.1)] lower than 0.7 micron, ii) no more than 50% of said particles have a volume diameter [d(v,0.5)] comprised between 1.6 micron and 1.9 micron; and iii) at least 90% of said particles have a volume diameter lower than 4.7 micron, and wherein said particles have a particle size span, defined as [d(v,0.9) - d(v,0.1)]/d(v,0.5), comprised between 1.5 and 2.2 micron, whereby said volume diameter is determined by laser diffraction.

In a second embodiment, the invention relates to the process for the preparation of the aforementioned formulation.

In a third embodiment, the invention also relates to a vial filled with the claimed free formulation.

In a fourth embodiment, the invention refers to the claimed formulation for use for the prevention and/or treatment of an inflammatory or obstructive airways disease such as asthma or chronic obstructive pulmonary disease (COPD).
or chronic obstructive pulmonary disease (COPD), which comprises administration by inhalation of an effective amount of the formulation of the invention.

In a sixth embodiment, the invention refers to the use of the claimed formulation in the manufacture of a medicament for the prevention and/or treatment of an inflammatory or obstructive airways disease such as asthma or chronic obstructive pulmonary disease (COPD).

In a seventh embodiment, the invention refers to a kit comprising:
a) the formulation of the invention filled in a unit-dose vial; and
b) a nebulizer.

### DEFINITIONS

The terms "active drug", "active ingredient", "active" and "active substance", "active compound" and "therapeutic agent" are used as synonymous.

The term "corticosteroids" refers to class of active ingredients having a hydrogenated cyclopentoperhydrophenanthrene ring system endowed with an anti-inflammatory activity.

The term "propellant free" indicates that the formulation is not delivered in admixture with any of the commonly used aerosol propellants, such as hydrofluorocarbons, hydrocarbons, compressed gases, and the like.

The expression "insoluble or poorly water soluble" refers to an active ingredient having a solubility in water as defined in the European Pharmacopoeia Ed. 4th, 2003, page 2891.

By the term "nebulisation", it is meant the generation of very fine liquid droplets for inhalation to the lungs by means of suitable devices called nebulisers.

By the term "sterile" it is meant a product which meets the criteria of sterility according to the European Pharmacopoeia (Ph. Eur. 1998, Chapters 2.6.1 and 5.1.1). Further regulations for sterility of the final product include the US Pharmacopoeia 23/NF18, 1995, pp. 1686-1690 and 1963-1975.

In the present application, the particle size is quantified by measuring a characteristic equivalent sphere diameter, known as volume diameter by laser diffraction. The volume diameter (VD) is related to the mass diameter (MD) by the density of the particles (assuming a size independent density for the particles). Particle size distribution is described by: i) the volume median diameter (VMD) which corresponds to the diameter of 50 percent by volume of the particles [d(v,0.5)], and ii) the VD (MD) in micron of 10% and 90% of the particles [d(v,0.1) and d(v,0.9)].

Upon aerosolisation, the particle size is expressed as mass aerodynamic diameter (MAD) and the particle size distribution as mass median aerodynamic diameter (MMAD). The MAD indicates the capability of the particles of being transported suspended in an air stream. The MMAD corresponds to the mass aerodynamic diameter of 50 percent by weight of the particles.

In the context of the suspension formulations, the expression "physically stable" refers to formulations which exhibit substantially no growth in particle size or change in crystal morphology of the active ingredient over a prolonged period, are readily redispersible, and upon redispersion, do not flocculate so quickly as to prevent reproducing dosing of the active ingredient.

The expression "respirable fraction" refers to an index of the percentage of active particles which would reach the deep lungs in a patient.

The respirable fraction, also termed fine particle fraction, is evaluated using a suitable *in vitro* apparata such as Multistage Cascade Impactor or Multi Stage Liquid Impinger (MLSI) according to procedures reported in common Pharmacopoeias.

It is calculated by the ratio between the delivered dose and the fine particle mass (formerly fine particle dose).

The emitted dose is calculated from the amount of active ingredient collected on the filter; the delivered dose is calculated from the cumulative deposition in the apparatus, while the fine particle mass is calculated from the deposition on Stages 3 (S3) to filter (AF) corresponding to particles <6.4 microns.

The term "prevention" means an approach for reducing the risk of onset of a disease.

The term "treatment" means an approach for obtaining beneficial or desired results, including clinical results. Beneficial or desired clinical results can include, but are not limited to, alleviation or amelioration of one or more symptoms or conditions, diminishment of extent of disease, stabilized (i. e. not worsening) state of disease, preventing spread of disease, delay or slowing of disease progression, amelioration or palliation of the disease state, and remission (whether partial or total), whether detectable or undetectable. The term can also mean prolonging survival as compared to expected survival if not receiving treatment.

### DETAILED DESCRIPTION OF THE INVENTION

The propellant-free sterile pharmaceutical formulation of the invention for administration through nebulisation comprises micronized crystalline particles of beclometasone dipropionate monohydrated suspended in an aqueous phase.

The monohydrated form is commonly distinguished from the anhydrous form by their powder X-ray diffractometry pattern.

The suspended micronized particles of the drug are characterized by a selected, narrow, and well defined particle size distribution in such a way that: i) no more than 10% of said suspended particles have a volume diameter [d(v,0.1)] lower than 0.7 micron ii) no more than 50% of said particles have a volume diameter [d(v,0.5)] comprised between 1.6 micron and 1.9 micron, preferably between 1.6 and 1.8 micron; and iii) at least 90% of said particles [d(v,0.9)] have a volume diameter equal to or lower than 4.7 micron, preferably equal to or lower than 4.0 micron.

The suspended particles should also have a width of the particle size distribution expressed as a span comprised between 1.5 and 2.2, preferably between 1.5 and 2.1, more preferably between 1.8 and 2.1.According the Chew et al J Pharm Pharmceut Sci 2002, 5, 162-168, the span corresponds to [d(v,0.9) - d(v,0.1)]/d(v,0.5).

Advantageously, substantially all the suspended particles have a volume diameter comprised between 6 and 0.4 micron, preferably between 5.5 and 0.45 micron.

The particle size of the active substance is determined by measuring the characteristic equivalent sphere diameter, known as volume diameter, by laser diffraction as described above, preferably using a Malvern apparatus available from Malvern Instruments Ltd.

The formulation according to the invention exhibits a homogenous distribution of the particles as well as a higher level of physical stability in comparison to the formulations of the prior art, as the particles sediment more slowly and are less liable to form agglomerates.

Typically the aqueous phase comprises a pharmaceutically acceptable excipient selected from the group consisting of wetting agents, isotonicity agents, and optionally stabilisers and/or buffers for adjusting the pH.

The wetting agent may be selected from the group consisting of polysorbate 20, polysorbate 80, and sorbitan monolaurate. The isotonicity agent is typically sodium chloride.

If present, the stabilizer might be selected from complexing agents such as ethylenediaminetetraacetic acid (EDTA) or a salt thereof, i.e. the disodium salt, while the buffer might be a citric or a phosphate buffer.

Advantageously the pH of the formulation is comprised between 3.5 and 6.0.

Due to the particle size characteristics, and in particular due to the high surface area that is inversely proportional to the particle size distribution, the corticosteroid particles turn out to be more wettable and easy to be dispersed, and hence the formulation of the invention could be prepared using common procedures known in the art. For example, the preparation may be carried out in a turboemulsifier, an apparatus commonly used in the art.

Typically, the process of preparation comprises the following steps:
a) an aqueous solution comprising suitable the suitable excipients is prepared in a suitable tank, then transferred to a turboemulsifier provided with a turbine adapted for homogenising the suspension, and optionally with a mechanical agitator;
b) the active ingredient in the form of a micronised particles is added to the aqueous solution;
c) the suspended particles of the active ingredient are homogenised by stirring.

Advantageously, the preparation of the formulation may be performed using a turboemulsifier according to the conditions described in WO 00/25746, or preferably under vacuum according to the conditions described in WO 03/086374.

The formulation of the invention must be sterile. Said sterility requirement could be fulfilled in different ways.

For example, the formulation may be sterilised by subjecting the aqueous suspension obtained from a non-sterile micronized active ingredient to steam sterilization according to the conditions disclosed in EP 1599233.

Alternatively, the formulation can be prepared under aseptic conditions starting from micronised sterile particles of the corticosteroid.

In turn, the micronised sterile corticosteroid may be prepared by subjecting first the powder to micronisation, then to sterilization by irradiation or dry heating.

Preferably, it shall be sterilised by treatment with gamma rays under the conditions reported in WO 00/25746. Otherwise, the micronised corticosteroid may be sterilized by dry heating according to the conditions disclosed in EP 1032396, a process also known as tyndallization.

The micronised sterile corticosteroid may also be prepared by subjecting first the powder to sterilisation by means of irradiation with gamma or beta rays, and then to sterile micronisation, according to the teaching of WO 2007/062685.

The micronisation step is usually carried out by grinding using a conventional fluid energy mill such as the jet mill apparatus. Depending on the type of the apparatus and size of the batch, the person skilled in the art shall suitably adjust the milling parameters such as the operating pressure and the feeding rate to achieve the desired particle size.

Preferably the micronisation is carried out with the exclusion of moisture, more preferably using an inert gas such as nitrogen.

Advantageously, the micronized particles of the corticosteroid have a specific surface area comprised between 5.5 and 9.0 m²/g, preferably between 6.5 and 8.0 m²/g. The Specific Surface Area is determined by Brunauer-Emmett-Teller (BET) nitrogen adsorption method according to a procedure known in the art.

In a further embodiment, the micronised sterile particles of the corticosteroid could be obtained in the desired particle size by crystallization, followed by micronisation, both under aseptic conditions.

The concentration of BDP monohydrate is 0.04% w/v.

The formulation of the invention could also comprise one or more additional active ingredients suspended or dissolved in the aqueous phase.

Further active ingredients that could advantageously be used are those useful for the treatment of respiratory diseases, for example short-acting or long- acting beta₂-agonists such as salbutamol (albuterol), fenoterol, salmeterol and formoterol or salts thereof or anticholinergics drugs such as ipratropium bromide.

In a preferred embodiment, the formulation may comprise salbutamol in form of sulfate salt dissolved in the aqueous phase.

The formulation of the invention may be distributed in suitable containers such as multidose vials or, preferably, unit-dose vials for single dosage administration. Said vials may be made of glass or plastic materials. For example plastic materials for preparing the unit-dose vials include, but are not limited to, low density polyethylene, high density polyethylene, polypropylene and polyesters.

The unit-dose vials may be pre-sterilised or, preferably, may be aseptically filled using the "blow, fill and seal" technology.

Advantageously, the unit-dose vials may have a volume of 1 ml, 2 ml, 2.5 ml, 3 ml, 4 ml or 5 ml, preferably of 2 ml.

As a consequence, this invention also relates a vial filled with the formulation of the invention.

The formulations of the invention are intended for administration by nebulisation using suitable apparatus known as nebulisers.

Any nebuliser can be used with the formulation of the invention.

Said nebulisers may produce the nebulised droplets by any method known to those skilled in the art, including, but not limited to, compressed air (jet), ultrasonic waves, or vibration.

Typically the formulation is administered using jet nebulisers coupled with suitable compressors like for example LC PLUS^{®} or LC Sprint^{®} (Pari GmbH, Germany).

Therefore the invention is also directed to a kit comprising the pharmaceutical formulation provided herein filled in a unit-dose vial and a nebulizer.

Administration of the formulation of the invention may be indicated for the prevention and/or treatment of a wide range of conditions including respiratory disorders such as chronic obstructive pulmonary disease (COPD) and asthma of all types.

### EXAMPLES

### EXAMPLE 1: Sterile suspension formulation comprising micronised anhydrous BDP as active ingredient

The formulation according to the invention was prepared starting from a suitable micronised anhydrous BDP made sterile by gamma-irradiation at 3.17 KGy as described in WO 00/25746.

The formulation was distributed in unit-dose vials using the "blow, fill and seal" technology.

Its composition is reported in Table 1

**Table 1**

| Ingredients | Total quantity of preparation | Quantity per pharmaceutical unit |
|---|---|---|
| Sterile micronised BDP | 0.6 kg | (0.80 mg) |
| Polysorbate (Tween) 20 | 1.5 kg | (2.0 mg) |
| Sorbitan monolaurate | 0.3 kg | (0.40 mg) |
| Sodium chloride | 13.5 kg | (18.0 mg) |
| Water for injection q. s. for | 1500 1 | (2.0 ml) |

The nebulisation efficiency was evaluated using the LC Sprint^{®} nebulizer (Pari GmbH, Germany) in a Multi Stage Liquid Impinger (MLSI) according to procedures reported in the European Pharmacopoeia.

The respirable fraction of BDP turned out to be of about 24% with a MMAD of the particles of 7.5 micron (GSD¹⁾ ± 1.8).

### ¹⁾ GSD is the geometric standard deviation

### EXAMPLE 2: Particle-size analysis of preparations obtained according to example 1

The dimensional characteristics of the particles were evaluated by using a Malvern apparatus and by microscopy.

This type of test exploits the diffraction of a laser beam by the particles to determine the size distribution of the particles in suspension. The parameter considered is the volumetric diameter in µm of 10%, 50% and 90% of the particles, expressed as d(v,0.1), d(v,0.5) and d(v,0.9) respectively, which is determined by assuming that the particles have a geometrical shape equivalent to a sphere. The particle size span, defined as d(v,0.9) - d(v,0.1)]/d(v,0.5), is also calculated.

The results are reported in Table 2 for the purpose of comparison with those relating to a suspension obtained as described in WO 03/086347.

**Table 2**

| Particle size (µm, Malvern) | BDP (according to the invention) | BDP (according to WO 03/086347) |
|---|---|---|
| d (v,0.1) | 0.67 | 0.78 |
| d (v,0.5) | 1.62 | 2.97 |
| d (v,0.9) | 3.99 | 7.88 |
| d(v,0.9) - d(v,0.1]/d(v,0.5) | 2.05 | 2.39 |

As it can be appreciated the formulation according to the invention exhibits a narrower particle-size distribution.

### EXAMPLE 3: Sterile suspension formulation comprising micronised anhydrous BDP and salbutamol as active ingredients

The formulation was prepared as described in Example 1 and distributed in unit-dose vials using the "blow, fill and seal" technology.

Its composition is reported in Table 3

**Table 3**

| Ingredients | Total quantity of preparation | Quantity per pharmaceutical unit |
|---|---|---|
| Sterile micronised BDP | 0.6 kg | (0.80 mg) |
| Salbutamol sulphate | 1.446 kg | (1.928 mg) |
| Polysorbate (Tween) 20 | 1.5 kg | (2.0 mg) |
| Sorbitan monolaurate | 0.3 kg | (0.4 mg) |
| Sodium chloride | 13.5 kg | (18.0 mg) |
| Water for injection q. s. for | 1500 1 | (2.0 ml) |

The nebulisation efficiency was evaluated using the LC Sprint^{®} nebulizer (Pari GmbH, Germany) in a Multi Stage Liquid Impinger (MLSI) according to procedures reported in the European Pharmacopoeia.

The respirable fraction of BDP turned out to be of about 24%, while that of salbutamol turned out be of about 36%.

The MMAD of BDP particles is of 6.7 micron (GSD ± 2.0) and the MMAD of salbutamol particles is 5.5 micron (GSD ± 2.2).

## Claims

1. A propellant-free sterile pharmaceutical formulation for use in the prevention and/or treatment of a respiratory disease selected from asthma and chronic obstructive pulmonary disease by nebulisation to the lungs, said formulation comprising micronized particles of beclometasone dipropionate monohydrate suspended in an aqueous solution at a concentration of 0.04% w/v, wherein i) no more than 10% of said suspended particles have a volume diameter [d(v,0.1 )] lower than 0.7 micron, ii) no more than 50% of said particles have a volume diameter [d(v,0.5)] comprised between 1.6 micron and 1.9 micron; and iii) at least 90% of said particles [d(v,0.9)] have a volume diameter equal to or lower than 4.7 micron, and wherein said particles have a particle size span, defined as [d(v,0.9) - d(v,0.1)]/d(v,0.5), comprised between 1.5 and 2.2, and whereby said volume diameter is determined by laser diffraction.

2. The formulation according to claim 1, wherein no more than 50% of the suspended particles have a volume diameter comprised between 1.6 and 1.8.

3. The formulation according to claim 1 or 2, wherein at least 90% of the suspended particles have a volume diameter equal to or lower than 4.0 micron.

4. The formulation according to claim 3, wherein the particle size span is comprised between 1.5 and 2.1.

5. The formulation according to any one of claims 1 to 4, wherein the specific surface area of the micronized particles of beclometasone dipropionate monohydrate is comprised between 5.5 and 9.0 m²/g.

6. The formulation according to claim 5, wherein the specific surface area is comprised between 6.5 and 8.0 m²/g.

7. The formulation according to any of the preceding claims, wherein the aqueous solution comprises one or more pharmaceutically acceptable excipients selected from the group consisting of wetting agent, an isotonicity agent, and optionally a stabiliser and/or a buffer for adjusting the pH.

8. The formulation according to claim 7, wherein the wetting agent is selected from the group consisting of polysorbate 20, polysorbate 80, and sorbitan monolaurate.

9. The formulation according to claim 7 or 8, wherein the isotonicity agent is sodium chloride.

10. The formulation according to any one of the preceding claims further comprising one or more additional active ingredients suspended or dissolved in the aqueous phase.

11. The formulation according to claim 10, wherein the additional active ingredient is salbutamol sulfate.

12. The formulation according to any one of claims 1 to 11, wherein the micronized particles of beclometasone dipropionate monohydrate are sterilised by gamma-irradiation or dry heating.

13. A process for preparing the sterile formulation of claim 1-12, comprising the steps of:
i) preparing an aqueous solution in a suitable tank;
ii) transferring it to a turboemulsifier provided with a turbine adapted for homogenizing a suspension, and optionally a mechanical agitator;
iii) adding the micronized particles of beclometasone dipropionate monohydrate to the aqueous solution to form an aqueous suspension; and
iv) homogenizing the suspended particles by stirring.

14. The process according to claim 13, wherein the aqueous suspension of step iii) is subjected to steam sterilization.

15. A vial filled with a formulation according to any one of claims 1 to 12.

16. A kit comprising:
(a) a formulation according to any one of claims 1 to 12 filled in a unit-dose vial; and
(b) a nebulizer.

## Patentansprüche

1. Treibmittelfreie sterile pharmazeutische Formulierung zur Verwendung in der Vorbeugung und/oder Behandlung einer Atemwegserkrankung, ausgewählt aus Asthma und chronischobstruktiver Lungenerkrankung, durch Verneblung in die Lunge, wobei die Formulierung umfasst: mikronisierte Partikel von Beclometasondipropionat-Monohydrat, suspendiert in einer wässrigen Lösung bei einer Konzentration von 0,04% G/V, wobei i) nicht mehr als 10% der suspendierten Partikel einen Volumendurchmesser [d(V,0,1)] von weniger als 0,7 Mikrometer aufweisen, ii) nicht mehr als 50% der Partikel einen Volumendurchmesser [d(V,0,5)], der von einem Bereich zwischen 1,6 Mikrometer und 1,9 Mikrometer umfasst ist, aufweisen; sowie iii) wenigstens 90% der Partikel [d(V,0,9)] einen Volumendurchmesser von gleich oder weniger als 4,7 Mikrometer aufweisen, und wobei die Partikel eine Partikelgrößenspanne, definiert als [d(V,0,9) - d(V,0,1)] / d(V,0,5), die von dem Bereich zwischen 1,5 und 2,2 umfasst ist, aufweisen, und wobei der Volumendurchmesser durch Laserbeugung bestimmt wird.

2. Formulierung gemäß Anspruch 1, wobei nicht mehr als 50% der suspendierten Partikel einen Volumendurchmesser, der von dem Bereich zwischen 1,6 und 1,8 umfasst ist, aufweisen.

3. Formulierung gemäß Anspruch 1 oder 2, wobei wenigstens 90% der suspendierten Partikel einen Volumendurchmesser aufweisen, der gleich oder geringer als 4,0 Mikrometer ist.

4. Formulierung gemäß Anspruch 3, wobei die Partikelgrößenspanne von dem Bereich zwischen 1,5 und 2,1 umfasst ist.

5. Formulierung gemäß einem der Ansprüche 1 bis 4, wobei die spezifische Oberfläche der mikronisierten Partikel von Beclometasondipropionat-Monohydrat von dem Bereich zwischen 5,5 und 9,0 m²/g umfasst ist.

6. Formulierung gemäß Anspruch 5, wobei die spezifische Oberfläche von dem Bereich zwischen 6,5 und 8,0 m²/g umfasst ist.

7. Formulierung gemäß einem der vorangehenden Ansprüche, wobei die wässrige Lösung ein pharmazeutisch annehmbares Exzipiens oder mehrere pharmazeutisch annehmbare Exzipienzien umfasst, ausgewählt aus der Gruppe, umfassend aus einem Benetzungsmittel, einem Isotonizitätsmittel und gegebenenfalls einem Stabilisator und/oder einem Puffer zur Einstellung des pH.

8. Formulierung gemäß Anspruch 7, wobei das Benetzungsmittel ausgewählt ist aus der Gruppe, bestehend aus Polysorbat 20, Polysorbat 80 und Sorbitanmonolaurat.

9. Formulierung gemäß Anspruch 7 oder 8, wobei das Isotonizitätsmittel Natriumchlorid ist.

10. Formulierung gemäß einem der vorangehenden Ansprüche, des Weiteren umfassend einen oder mehrere zusätzliche(n) Wirkstoff(e), der/die in der wässrigen Phase suspendiert oder gelöst ist/sind.

11. Formulierung gemäß Anspruch 10, wobei der zusätzliche Wirkstoff Salbutamolsulfat ist.

12. Formulierung gemäß einem der Ansprüche 1 bis 11, wobei die mikronisierten Partikel von Beclometasondipropionat-Monohydrat durch gamma-Strahlung oder trockenes Erhitzen sterilisiert werden.

13. Verfahren zur Herstellung der sterilen Formulierung gemäß Anspruch 1-12, umfassend die folgenden Schritte:
i) Herstellen einer wässrigen Lösung in einem geeigneten Tank;
ii) Übertragen desselben zu einem Turboemulgiergerät, das mit einer Turbine, die zum Homogenisieren einer Suspension angepasst ist, und gegebenenfalls einem mechanischen Rührer ausgestattet;
iii) Zugeben der mikronisierten Partikel von Beclometasondipropionat-Monohydrat zu der wässrigen Lösung unter Bildung einer wässrigen Suspension; und
iv) Homogenisieren der suspendierten Partikel durch Rühren.

14. Verfahren gemäß Anspruch 13, wobei die wässrige Suspension von Schritt iii) einer Dampfsterilisation unterworfen wird.

15. Phiole, gefüllt mit einer Formulierung gemäß einem der Ansprüche 1 bis 12.

16. Kit, umfassend:
(a) eine Formulierung gemäß einem der Ansprüche 1 bis 12, abgefüllt in einer Einheitsdosisphiole; sowie
(b) einen Vernebler.

## Revendications

1. Formulation pharmaceutique stérile sans gaz propulseur pour une utilisation dans la prévention et/ou le traitement d'une maladie respiratoire choisie parmi l'asthme et la broncho-pneumopathie chronique obstructive par nébulisation aux poumons, ladite formulation comprenant des particules micronisées de dipropionate de béclométasone monohydraté en suspension dans une solution aqueuse à un concentration de 0,04 % en p/v, dans laquelle i) pas plus de 10 % desdites particules en suspension ont un diamètre en volume [d(v,0,1)] inférieur à 0,7 micron, ii) pas plus de 50 % desdites particules ont un diamètre en volume [d(v,0,5)] compris entre 1,6 micron et 1,9 micron ; et iii) au moins 90 % desdites particules [d(v,0,9)] ont un diamètre en volume inférieur ou égal à 4,7 microns, et dans laquelle lesdites particules ont une étendue granulométrique, définie comme étant [d(v,0,9) - d(v,0,1)]/d(v,0,5), comprise entre 1,5 et 2,2, et par quoi ledit diamètre en volume est déterminé par diffraction laser.

2. Formulation selon la revendication 1, dans laquelle pas plus de 50 % des particules en suspension ont un diamètre en volume compris entre 1,6 et 1,8.

3. Formulation selon la revendication 1 ou 2, dans laquelle au moins 90 % des particules en suspension ont un diamètre en volume inférieur ou égal à 4,0 microns.

4. Formulation selon la revendication 3, dans laquelle l'étendue granulométrique est comprise entre 1,5 et 2,1.

5. Formulation selon l'une quelconque des revendications 1 à 4, dans laquelle la surface spécifique des particules micronisées de dipropionate de béclométasone monohydraté est comprise entre 5,5 et 9,0 m²/g.

6. Formulation selon la revendication 5, dans laquelle la surface spécifique est comprise entre 6,5 et 8,0 m²/g.

7. Formulation selon l'une quelconque des revendications précédentes, dans laquelle la solution aqueuse comprend un ou plusieurs excipients pharmaceutiquement acceptables choisis dans le groupe constitué par un agent mouillant, un agent d'isotonicité, et éventuellement un stabilisant et/ou un tampon pour ajuster le pH.

8. Formulation selon la revendication 7, dans laquelle l'agent mouillant est choisi dans le groupe constitué par le polysorbate 20, le polysorbate 80 et le monolaurate de sorbitan.

9. Formulation selon la revendication 7 ou 8, dans laquelle l'agent d'isotonicité est le chlorure de sodium.

10. Formulation selon l'une quelconque des revendications précédentes, comprenant en outre un ou plusieurs principes actifs supplémentaires en suspension ou dissous dans la phase aqueuse.

11. Formulation selon la revendication 10, dans laquelle le principe actif supplémentaire est le sulfate de salbutamol.

12. Formulation selon l'une quelconque des revendications 1 à 11, dans laquelle les particules micronisées de dipropionate de béclométasone monohydraté sont stérilisés par irradiation aux rayons gamma ou chauffage à sec.

13. Procédé de préparation de la formulation stérile selon la revendication 1 à 12, comprenant les étapes de :
i) préparation d'une solution aqueuse dans un réservoir approprié ;
ii) transfert de celle-ci dans un turbo-émulseur muni d'une turbine conçue pour l'homogénéisation d'une suspension, et éventuellement d'un agitateur mécanique ;
iii) ajout des particules micronisées de dipropionate de béclométasone monohydraté à la solution aqueuse pour former une suspension aqueuse ;
et
iv) homogénéisation des particules en suspension par agitation.

14. Procédé selon la revendication 13, dans lequel la suspension aqueuse de l'étape iii) est soumise à une stérilisation à la vapeur.

15. Flacon rempli d'une formulation selon l'une quelconque des revendications 1 à 12.

16. Kit comprenant:
(a) une formulation selon l'une quelconque des revendications 1 à 12 remplie dans un flacon unidose ; et
(b) un nébulisateur.
